# EUROPEAN PATENT APPLICATION

(11) **EP 4 212 593 A1**
(43) Date of publication of application: **19.07.2023**
(21) Application number: 23151645.1
(22) Date of filing: 16.01.2023
(51) Int. Cl.: C09B 69/10, G01N 33/52, G01N 33/58

(54) **WATER-SOLUBLE PI-CONJUGATED FLUORESCENT 1,1´-BINAPHTHYL-BASED POLYMERS WITH TUNEABLE ABSORPTION**

(30) Priority: 17.01.2022 EP 22151720
(71) Applicant: Miltenyi Biotec B.V. & Co. KG, 51429 Bergisch Gladbach (DE)
(72) Inventor: Meineke, Dirk, 51429 Bergisch Gladbach (DE); Berndt, Daniel, 51429 Bergisch Gladbach (DE); Dose, Christian, 51429 Bergisch Gladbach (DE); Jennings, Travis, 51429 Bergisch Gladbach (DE)
(74) Representative: Kisters, Michael Marcus

(57) **Abstract**

The invention is directed to a conjugate having the general formula (I)

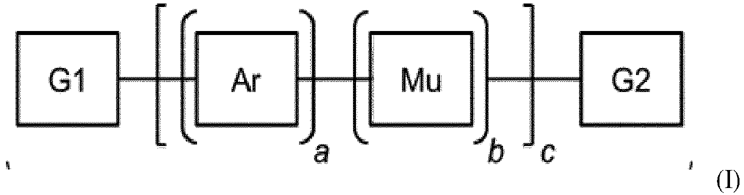

Wherein AR and MU are repeating units of a polymer
MU is a polymer modifying unit or band gap modifying unit that is evenly or randomly distributed along the polymer main chain,
G1 and G2 stand for hydrogen, halogen or an antigen recognizing moiety, with the provision that at least one of G1 or G2 is an antigen recognizing moiety,
a is 10 to 100 mol%,
b is 0 to 90 mol%
c is 1 to 10 000; with the provisio that a + b = 100 mol%
**characterized in that** AR is connected in the polymer chain via the 2,2' or 3,3' or 5,5' or 6,6' or 7,7' or 8,8' positions according to general formula (II)

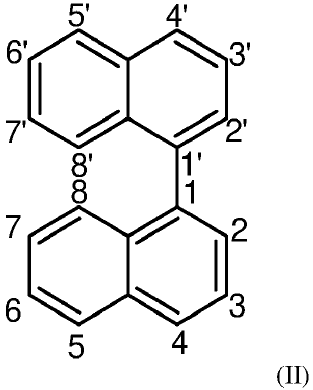

Wherein the remaining positions 2,2'; 3,3 ; 4,4'; 5,5'; 6,6'; 7,7' and 8,8' are substituted with same or different residues selected from the group consisting of H, SO₂CF₃, SO₂Rₐ, CF₃, CCl₃, CN, SO₃H, NO₂, NRₐR_{b}R_{c}⁺, CHO, CORa, CO₂Ra, COC1, CONRaRb, F, Cl, Br, I, Rₐ, ORₐ, SRₐ, OCORₐ, NRₐR_{b}, NHCORₐ, CCRₐ, aryl-, heteroaryl-, C₆H₄ORₐ or C₆H₄NRaRb, with Ra-c independently hydrogen, alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or a heteroaralkyl residue or (CH₂)x(OCH₂CH₂)yO(CH₂)zCH₃, wherein x is an integer from 0 to 20; y is an integer from 0 to 50 and z is an integer from 0 to 20

## Description

The present invention is to water-soluble fluorescent polymer dyes and their use in conjugates with antibodies for cell detection and analysis.

### BACKGROUND

Immunofluorescent labeling is important for the detailed analysis and specific isolation of target cells from a biological specimen in both, research and clinical applications. The sensitivity of the analysis mainly depends on the brightness of the fluorophores. Water-soluble π -conjugated polymers have been used as fluorophores with remarkable brightness and applied in biological applications.

Such water-soluble π-conjugated polymers are for example described in "Synthesis of Binaphthyl-Oligothiophene Copolymers with Emissions of Different Colors: Systematically Tuning the Photoluminescence of Conjugated Polymers" by K. Y. Musick, Q.-S. Hu, L. Pu, Macromolecules 1998, 31, 2933-2942.

Further, US20180009990A1 and US10604657B2 mention 4,4'-polymerized binaphthyl substituted with PEGs in the 2,2'positions. This references a binapthyl-based polymer in which the 4,4' bond is attached to neighboring monomers via polyparaethyniline (PPE) bond, which utilizes sp³ hybridized orbitals. The binaphthyl-based polymers disclosed in this invention are bound directly to neighboring co-monomers without ethyniline bonding, resulting in sp² hybridized bonding and fully-aromatic conjugated systems. Water solvated polymeric dyes and polymeric tandem dyes are provided. The polymeric dyes include a water solvated light harvesting multichromophore having a conjugated segment of aryl or heteroaryl co-monomers linked via covalent bonds, vinylene groups or ethynylene groups. The polymeric tandem dyes further include a signaling chromophore covalently linked to the multichromophore in energy-receiving proximity therewith.

The thus disclosed polymer dyes are expensive to manufacture and in particular the synthesis of 4,4'-functionalized binaphthyl monomers is laborious and expensive. Furthermore, special comonomers are required to use these polymer dyes with ultraviolet excitation light and and the resulting polymer dyes are highly complex. It is therefore an object of the invention to provide simple systems which give more flexibility in tuning the absorption and emission characteristics towards the UV region of the spectrum and which are also easier and cheaper to produce.

### SUMMARY OF THE INVENTION

pi-Conjugated polymers based on binaphthyl monomers allow tuning the absorption wavelength over an extremely wide range. The energy of the system is strikingly high because of to the unique structure of the binaphthyl monomers which consist of two twisted naphthyl units. In contrast to state-of-the-art polyfluorene and polyphenanthrene based polymers, this structural feature allows the design of polymers with absorption in the deep UV region.

The energy of the system can be precisely controlled by copolymerization of the binaphthyl monomers with suitable comonomers. Strategic choice of comonomers gives the ability to tune the polymer absorption band edge from the deep UV into the visible range of the spectrum.

Using the unique monomers and combining these with specific comonomers gives the control over the position of the absorption band in the spectrum to exactly match the desired excitation laser, thus providing a solution to the technical problem.

To use the pi-conjugated polymers as fluorescent probes have become a mainstay in the toolbox of fluorescence detection. The absorption band of a useful polymer needs to match the wavelength of the light source, e.g. lasers with 355 nm, 405 nm, 488 nm, 561 nm or 640 nm wavelength. Current polymers, however, are limited in their light absorption band to narrow regions of the violet, which in turn limits their usefulness. Novel pi-conjugated polymers with tuneable absorption band would allow the expansion of the arsenal of water-soluble polymeric dyes for biological applications, including the detection of analytes.

### BRIEF DESCRIPTION OF THE INVENTION

Accordingly, an object of the invention is a conjugate having the general formula (I) 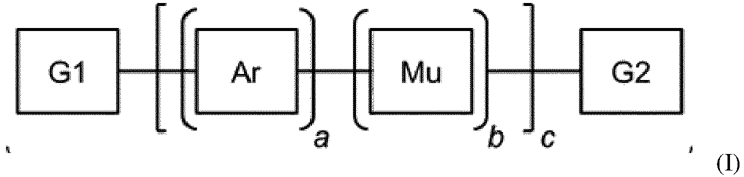
Wherein AR and MU are repeating units of a polymer
MU is a band gap modifying unit that is evenly or randomly distributed along the polymer main chain,
G1 and G2 stand for hydrogen, halogen or an antigen recognizing moiety, with the provision that at least one of G1 or G2 is an antigen recognizing moiety,
a is 10 to 100 mol%,
b is 0 to 90 mol%
c is 1 to 10 000; with the provisio that a + b = 100 mol%
**characterized in that** AR is connected in the polymer chain via the 2,2' or 3,3' or 5,5' or 6,6' or 7,7' or 8,8' positions according to general formula (II) 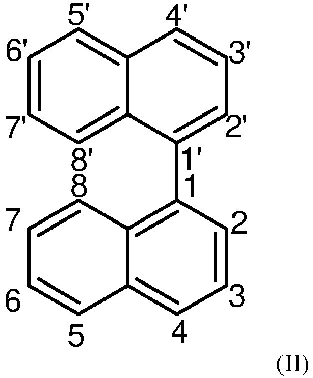
Wherein the remaining positions 2,2'; 3,3'; 4,4'; 5,5'; 6,6'; 7,7' and 8,8' are substituted with same or different residues selected from the group consisting of H, SO₂CF₃, SO₂Rₐ, CF₃, CCl₃, CN, SO₃H, NO₂, NRₐR_{b}R_{c}⁺, CHO, CORa, CO₂Ra, COCl, CONRaRb, F, Cl, Br, I, Rₐ, ORₐ, SRₐ, OCORₐ, NRₐR_{b}, NHCORₐ, CCRₐ, aryl-, heteroaryl-, C₆H₄ORₐ or C₆H₄NRaRb, with Ra-c independently hydrogen, alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or a heteroaralkyl residue or (CH₂)ₓ(OCH₂CH₂)_{y}O(CH₂)_{z}CH₃, wherein x is an integer from 0 to 20; y is an integer from 0 to 50 and z is an integer from 0 to 20.

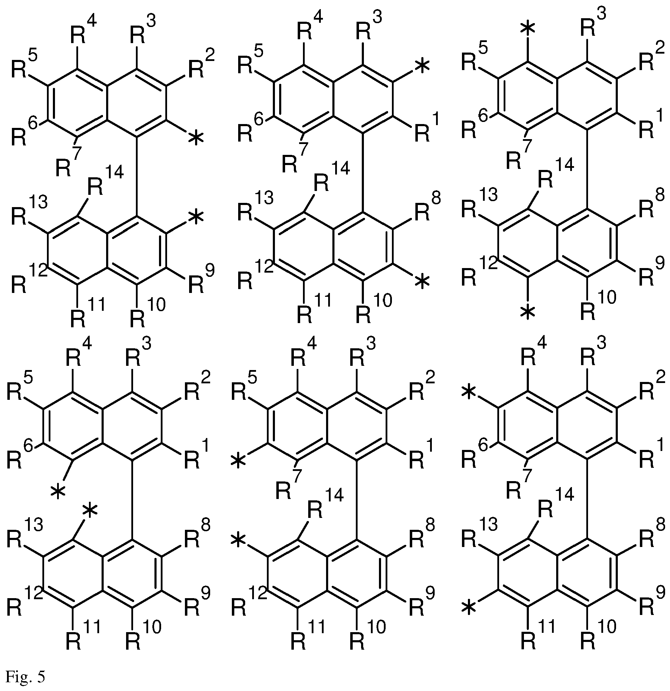

As can be seen from formula II, two positions out of 2,2'; 3,3'; 4,4'; 5,5'; 6,6'; 7,7' and 8,8' are needed for binding AR into the polymer chain. Accordingly, the term "remaining positions" refers to the 12 positions of AR that are not used for the binding of AR in the polymer chain and which are available for substitution with the residues as disclosed. Fig. 5 shows possible substitution patterns. The residues used for substitution may be same or different in each position (for example position 2 and 2' may be provided with the same or a different residue.

Optionally, two residues at the remaining positions may form a common cycloalkyl- or heterocycloalkyl ring system.

In a variant on the invention, at least two positions 2,2'; 3,3'; 4,4'; 5,5'; 6,6' 7,7' and 8,8' are substituted with residues according to general formula (IV) 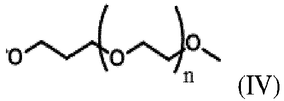 With n = 5 to 15

The conjugates of the invention are preferable water-soluble.

The term "AR is connected in the polymer chain via the 2,2' or 3,3' or 5,5' or 6,6' or 7,7' or 8,8' positions" refers to C-C bonds between the respective C atoms of the positions of AR with a C-atom of another AR unit, a G1 unit or an L unit.

Further object of the invention Is a method for detecting a target moiety in a sample of biological specimens with at least one conjugate as disclosed herein characterized by the steps
a) contacting the sample of biological specimens with at least one conjugate, thereby labeling the target moiety recognized by an antigen recognizing moiety with the conjugate;
b) exciting the labelled target moieties with light having a wavelength within the absorbance spectrum of the conjugate
c) detecting the labelled target moieties by detecting the fluorescence radiation emitted by the conjugate.

Yet another object of the invention is the use of the method in fluorescence microscopy, flow cytometry, fluorescence spectroscopy, cell separation, pathology or histology.

### BRIEF DESCRIPTION OF THE DRAWINGS

Fig. 1 shows the absorption (solid line) and emission (dashed line) spectra of water-soluble poly(binaphthyl) homopolymer **1.**
Fig. 2 shows the absorption (solid line) and emission (dashed line) spectra of water-soluble binaphthyl based phenylene copolymer **2.**
Fig. 3 shows the absorption (solid line) and emission (dashed line) spectra of water-soluble binaphthyl based bithiophene copolymer **3.**
Fig. 4 shows the flow cytometric analysis of human peripheral blood mononuclear cells (PBMCs) upon staining with anti-CD4 and anti-CD19 antibody conjugates of copolymer **2** on a commercially available flow cytometer
Fig. 5 shows substitution pattern of AR
Fig. 6 shows the conjugate of the invention comprising a polymeric part and an antigen recognizing moiety

### MU as bandgap modifying unit

MU is a bandgap modifying unit which causes a shift of the absorption band of the conjugate. To this end, MU comprises an aromatic system with at least one benzene or thiophene ring providing an conjugated pi system, optionally substituted with an electron withdrawing residue, an electron donating residue, sterically hindering groups, and/or residues enhancing water solubility.

More preferable, MU comprises an aromatic system with at least two fused, annulated or conjugated benzene or thiophene rings providing an conjugated pi system, optionally substituted with an electron withdrawing residue, an electron donating residue, sterically hindering groups, and/or residues enhancing water solubility.

As electron withdrawing residue, an electron donating residue, sterically hindering groups, and/or residues enhancing water solubility one or more residues like alkyl or ether (glycol) residues or halogen atoms or halogenated alkyl groups can be used.

The MU repeating units may evenly or randomly distributed in the conjugate of the invention. Insofar, MU has not only the function as band gap modifying unit, but also as polymer modifying unit.

Preferred residues for MU are as follows:

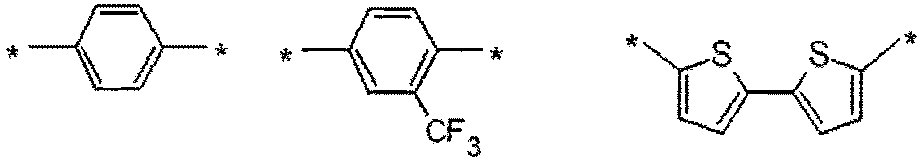

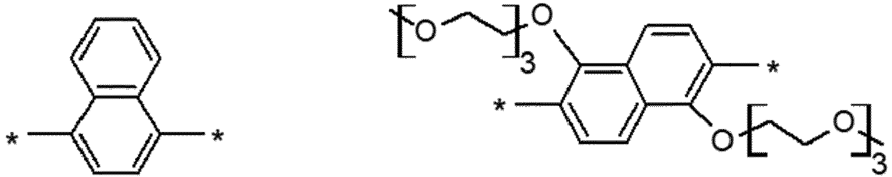

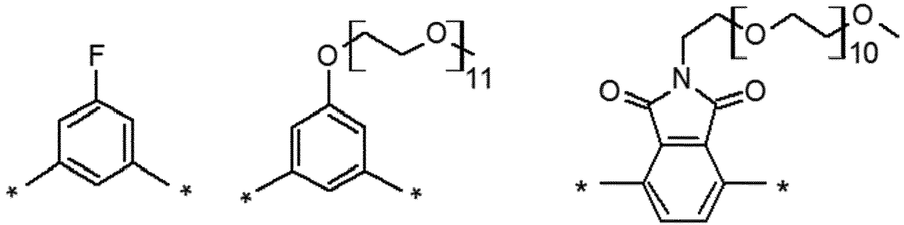

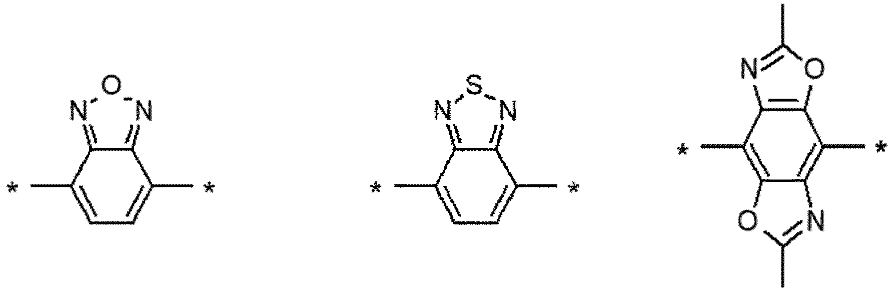

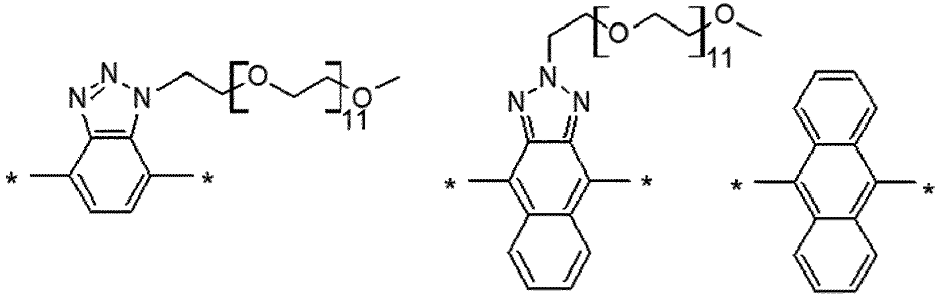

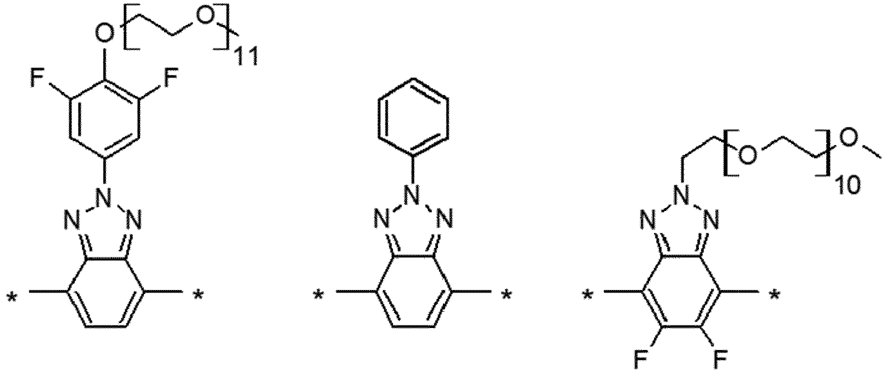

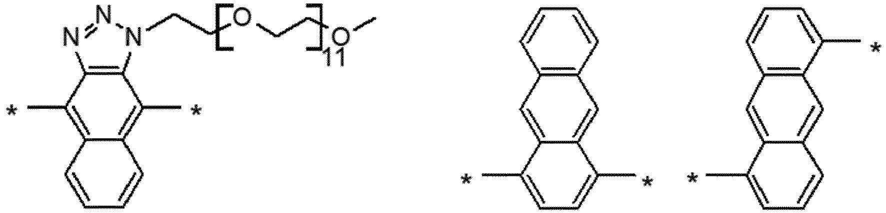

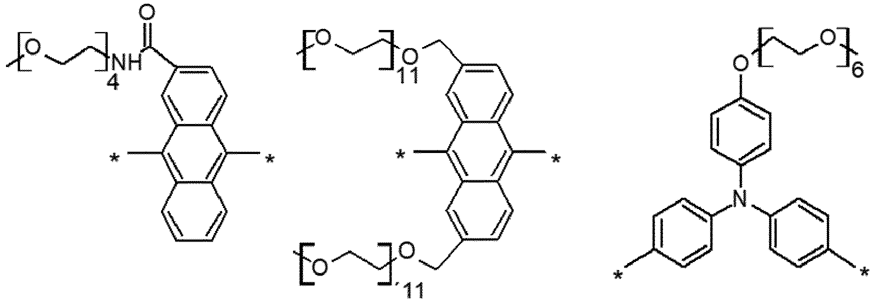

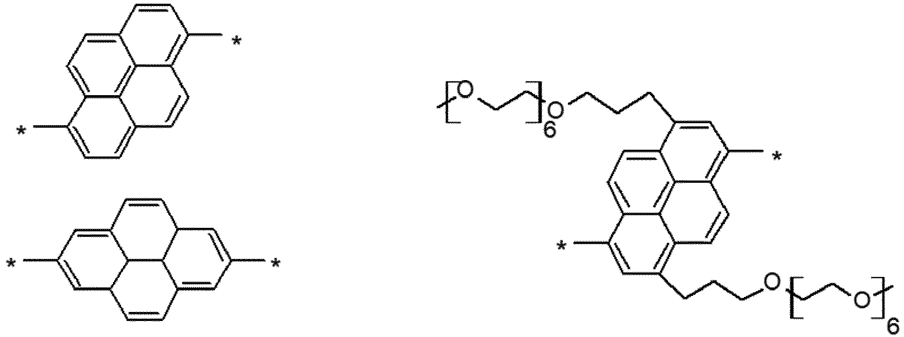

### Terminal groups G1 and G2

G1 and G2 are both independently chosen from the group of hydrogen, halogen or an antigen recognizing moiety. The antigen recognizing moiety could be selected from an antibody, an fragmented antibody, an fragmented antibody derivative, peptide/MHC-complexes, receptors for cell adhesion or costimulatory molecules, receptor ligands, antigens, hapten binders, avidin, streptavidin, aptamers, primers and ligase substrates, peptide/MHC complexes targeting TCR molecules, cell adhesion receptor molecules, receptors for costimulatory molecules or artificial engineered binding molecules.

### Linker group L

In a first embodiment of the invention, the MU unit located on the end of the conjugate is bond via a linker group L as shown in the following formula (III) 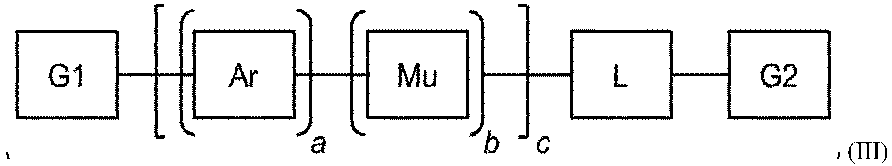 Wherein G1, AR, MU, G2, a, b and c have the same meaning as in formula (I) and wherein L represents a molecule comprising alkyl, aryl or heteroaryl groups connecting the polymer to G2.

L is an aryl or a heteroaryl group located at the ends of the polymer main chain and may be substituted with one or more pendant chains terminated with: i) a functional group selected from amine, carbamate, carboxylic acid, carboxylate, maleimide, activated ester, N-hydroxysuccinimidyl, hydrazine, hydrazide, hydrazine, azide, alkyne, aldehyde, thiol, and protected groups thereof for conjugation to a molecule or biomolecule; or ii) an attached organic fluorescent dye as energy acceptor of an energy transfer system, or iii) a biomolecule.

Preferable, L is selected from one or more of the following residues:

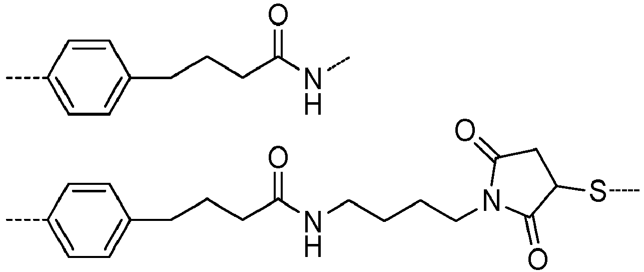

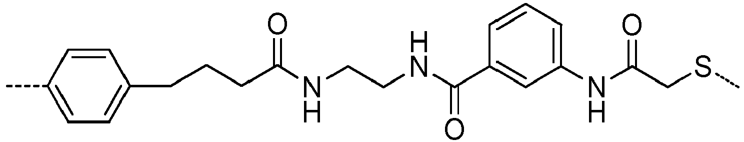

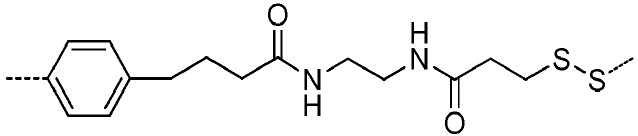

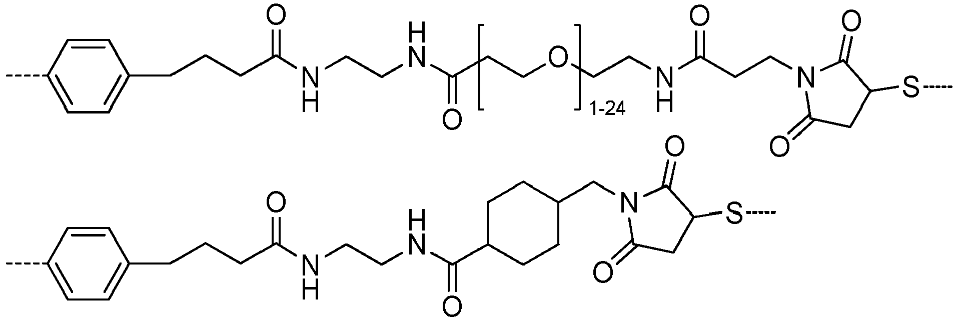

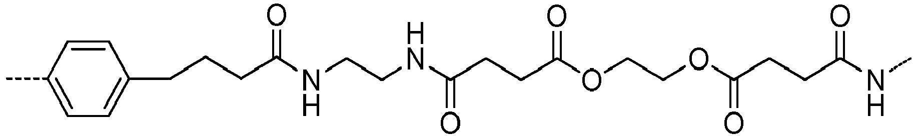

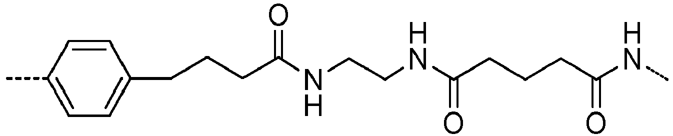

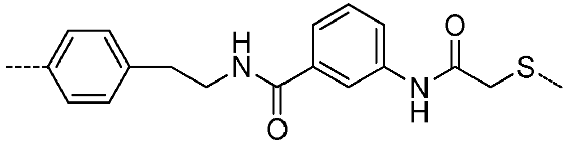

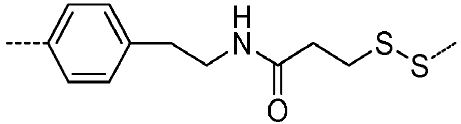

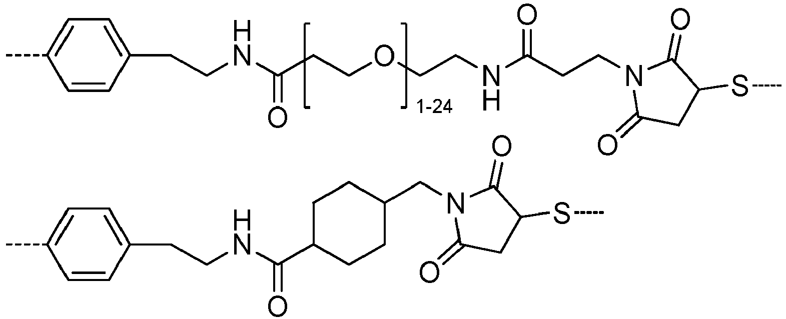

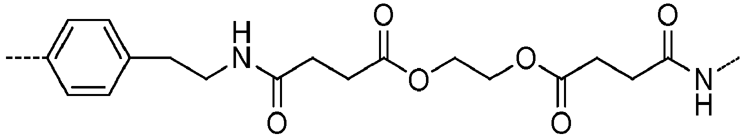

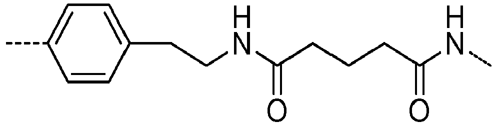

### Antigen recognizing moiety

The term "antigen recognizing moiety" refers to any kind of antibody, fragmented antibody or fragmented antibody derivatives, directed against the target moietiesexpressed on the biological specimens, like antigens expressed intracellular or extracellular on cells. The term relates to fully intact antibodies, fragmented antibody or fragmented antibody derivatives, e. g., Fab, Fab', F(ab')2, sdAb, scFv, di-scFv, nanobodies. Such fragmented antibody derivatives may be synthesized by recombinant procedures including covalent and non - covalent conjugates containing these kinds of molecules. Further examples of antigen recognizing moieties are peptide/MHC-complexes targeting TCR molecules, cell adhesion receptor molecules, receptors for costimulatory molecules, artificial engineered binding molecules, e.g., peptides or aptamers which target, e.g., cell surface molecules.

The conjugate used in the method of the invention may comprise up to 100, preferable 1-20 antigen recognizing moieties Y. The interaction of the antigen recognizing moiety with the target antigen can be of high or low affinity. Binding interactions of a single low - affinity antigen recognizing moiety is too low to provide a stable bond with the antigen. Low-affinity antigen recognizing moieties can be multimerized by conjugation to the enzymatically degradable spacer to furnish high avidity. When the spacer is enzymatically cleaved, the low-affinity antigen recognizing moieties will be monomerized which results in a complete removal of the fluorescent marker.

Preferable, the term "Antigen recognizing moiety" refers to an antibody directed against antigen expressed by the biological specimens (target cells) intracellular, like IL2, FoxP3, CD154, or extracellular, like CD19, CD3, CD14, CD4, CD, CD25, CD34, CD56, and CD133. The antigen recognizing moieties G1, G2, especially antibodies, can be coupled to CP through side chain amino or sulfhydryl groups. In some cases, the glycosidic side chain of the antibody can be oxidized by periodate resulting in aldehyde functional groups.

The antigen recognizing moiety can be covalently or non-covalently coupled. Methods for covalent or non-covalent conjugation are known by persons skilled in the art and the same as mentioned for conjugation of the fluorescent marker.

The method of the invention is especially useful for detection and/or isolation of specific cell types from complex mixtures and may comprise more than one sequential sequences of the steps a) - c). The method may use a variety of combinations of conjugates. For example, a conjugate may comprise antibodies specific for two different epitopes, like two different anti-CD34 antibodies. Different antigens may be addressed with different conjugates comprising different antibodies, for example, anti-CD4 and anti-CD8 for differentiation between two distinct T-cell-populations or anti-CD4 and anti-CD25 for determination of different cell subpopulations like regulatory T-cells.

### Cell Detection Methods

Targets labelled with the conjugate are detected by exciting either the fluorescent moiety FL or the backbone CP and analysing the resulting fluorescence signal. The wavelength of the excitation is usually selected according to the absorption maximum of the fluorescent moiety FL or CP and provided by LASER or LED sources as known in the art. If several different detection moieties FL are used for multiple colour/parameter detection, care should be taken to select fluorescent moieties having not overlapping absorption spectra, at least not overlapping absorption maxima. In case of fluorescent moieties the targets may be detected, e.g., under a fluorescence microscope, in a flow cytometer, a spectrofluorometer, or a fluorescence scanner. Light emitted by chemiluminescence can be detected by similar instrumentation omitting the excitation.

### Target Moiety

The target moiety to be detected with the method of the invention can be on any biological specimen, like tissues slices, cell aggregates, suspension cells, or adherent cells. The cells may be living or dead. Preferable, target moieties are antigens expressed intracellular or extracellular on biological specimen like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo Sapiens).

### Use of the Method

The method of the invention can be used for various applications in research, diagnostics and cell therapy, like in fluorescence microscopy, flow cytometer, fluorescence spectroscopy, cell separation, pathology or histology.

In a first variant of the invention, biological specimens like cells are detected for counting purposes i.e. to establish the amount of cells from a sample having a certain set of antigens recognized by the antigen recognizing moieties of the conjugate. In another variant, the biological specimens detected by the conjugate in step c) are separated from the sample by optical means, electrostatic forces, piezoelectric forces, mechanical separation or acoustic means.

In another variant of the invention, the location of the target moieties like antigens on the biological specimens recognized by the antigen recognizing moieties of the conjugate is determined. Such techniques are known as "Multi Epitope Ligand Cartography", "Chip-based Cytometry" or "Multiomyx" and are described, for example, in EP0810428, EP1181525, EP 1136822 or EP1224472. In this technology, cells are immobilized and contacted with antibodies coupled to fluorescent moiety. The antibodies are recognized by the respective antigens on the biological specimen (for example on a cell surface) and after removing the unbound marker and exciting the fluorescent moieties, the location of the antigen is detected by the fluorescence emission of the fluorescent moieties. In certain variants, instead of antibodies coupled to fluorescent moieties, antibodies coupled to moieties detectable for MALDI-Imaging or CyTOF can be used. The person skilled in the art is aware how to modify the technique based on fluorescent moiety to work with these detection moieties.

The location of the target moieties is achieved by a digital imaging device with a sufficient resolution and sensitivity in for the wavelength of the fluorescence radiation, the digital imaging device may be used with or without optical enlargement for example with a fluorescence microscope. The resulting images are stored on an appropriate storing device like a hard drive, for example in RAW, TIF, JPEG, or HDF5 format.

In order to detect different antigens, different antibody-conjugates having the same or different fluorescent moiety or antigen recognizing moiety can be provided. Since the parallel detection of fluorescence emission with different wavelengths is limited, the antibody-fluorochrome conjugates are utilized sequentially individually or in small groups (2-10) after the other.

In yet another variant of the method according to the invention, the biological specimens especially suspension cells of the sample are immobilized by trapping in microcavities or by adherence.

In general, the method of the invention can be performed in several variants. For example, the conjugate not recognized by a target moiety can be removed by washing for example with buffer before the target moiety labelled with the conjugate is detected.

In a variant of the invention, at least two conjugates are provided simultaneously or in subsequent staining sequences, wherein each antigen recognizing moiety recognizes different antigens. In an alternative variant, at least two conjugates can be provided to the sample simultaneously or in subsequent staining sequences, . In both cases, the labelled target moieties can be detected simultaneously or sequentially.

### EXAMPLES

By way of example, the following water-soluble binaphthyl based polymers 1-3 were synthesized and provided in position G1 with an antigen recognizing moiety

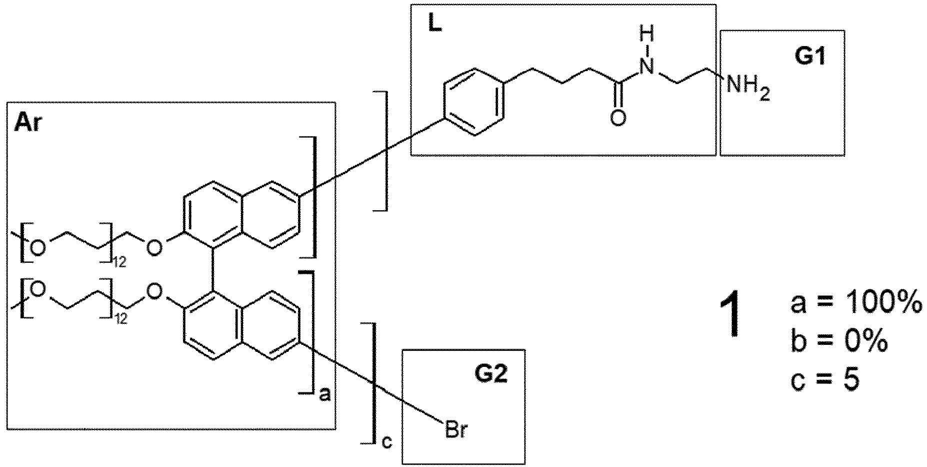

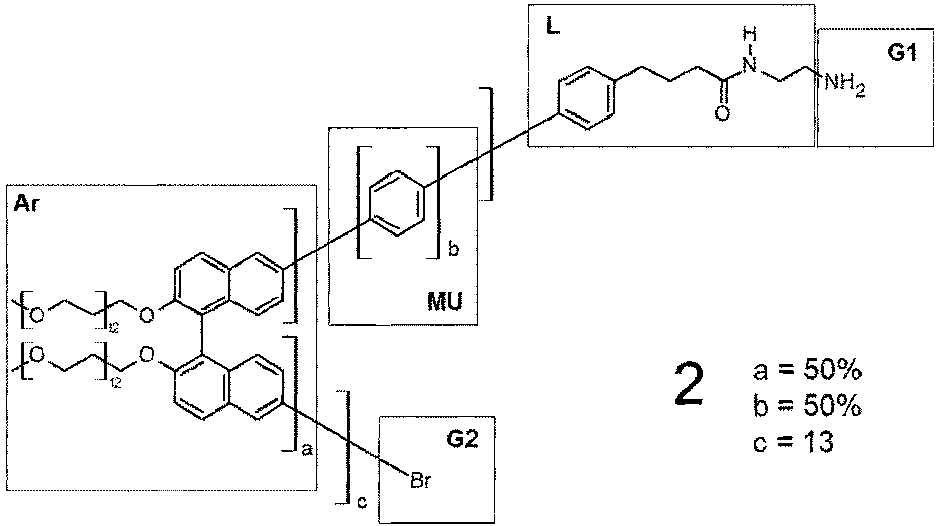

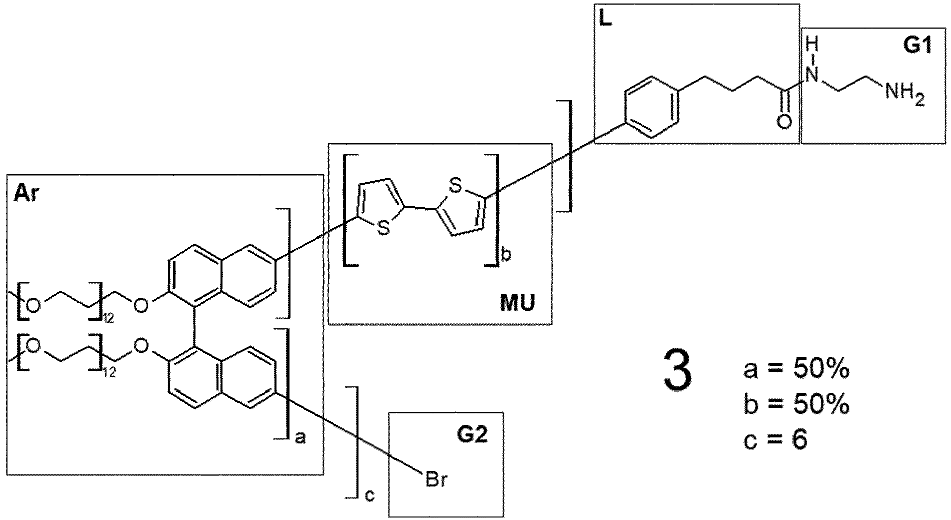

### Synthesis of building block M1

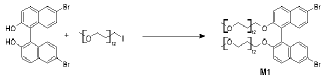

To a stirred solution of 6,6'-dibromo-[1,1'-binaphthalene]-2,2'-diol (3.00 g, 6 mmol) in acetone (42 mL), was added 34-iodo-2,5,8,11,14,17,20,23,26,29,32-undecaoxa tetratriacontane (12.6 g, 20 mmol), followed by K₂CO₃ (2.82 g, 20 mol), and the resulting reaction mixture was stirred at 70 °C for 16 h. The reaction mixture was filtered and concentrated under reduced pressure to dryness. The obtained crude material was diluted with water (20 mL) and extracted with ethyl acetate (3 x 30 mL). The combined organic phases were washed with brine (20 mL), dried over Na₂SO₄, filtered and concentrated under reduced pressure. The obtained crude residue was purified by silica column chromatography eluting with methanol/dichloromethane (5:95) giving a pale-yellow liquid. This compound was further purified by reverse phase chromatography, using eluting with acetonitrile/water (50:50). The compound was freeze dried to give pure compound **M1** (1.7 g, 17.6 %) as a pale-yellow liquid.

### Synthesis of building block M2

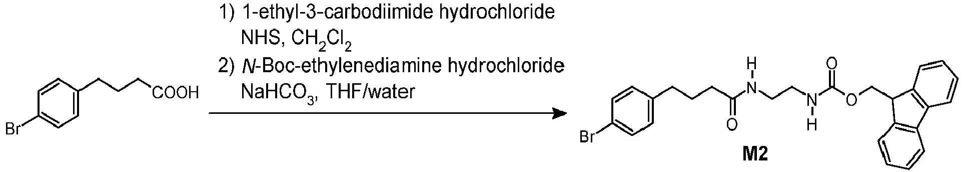

A stirred solution of 4-(4-bromophenyl)butanoic acid (2.00 g, 8.22 mmol) in dichloromethane (20 mL) was cooled to 0 °C, *N,N*-diisopropylethylamine (4.40 mL, 24.7 mmol) was added, followed by 3-(3-dimethylaminopropyl)carbodiimide hydrochloride (2.36 g, 12.3 mmol). The mixture was stirred for 10 min and *N*-hydroxysuccinimide (1.42 g, 12.3 mmol) was added. The reaction mixture was stirred for 16 h at room temperature. The solvents were removed under reduced pressure and saturated sodium hydrogencarbonate solution (50 mL) was added. The suspension was filtered and the residue was dried under reduced pressure to obtain 2.4 g of the crude product as an off white solid. The solid was washed with ethanol and dried under reduced pressure to yield the NHS ester as a white solid (1.24 g). The NHS-ester (2.00 g, 5.89 mmol) was dissolved with *N*-Boc-ethylenediamine hydrochloride (2.06 g, 6.48 mmol) in a mixture of tetrahydrofuran (40 mL) and water (20 mL) at 0 °C and sodium hydrogencarbonate (2.47 g, 29.5 mmol) was added. The reaction temperature was slowly raised to room temperature and the mixture was stirred for 7 h. The reaction mixture was concentrated under reduced pressure to remove the tetrahydrofuran, then water (100 mL) was added and the suspension was stirred for 15 min. The resulting solid was filtered off and dried under reduced pressure to give the product (2.0 g) as an off white solid. The solid was recrystallized from isopropyl alcohol (24 mL) giving compound **M2** as white solid (1.15 g, 38 %).

### Synthesis of binaphthyl homopolymer 1

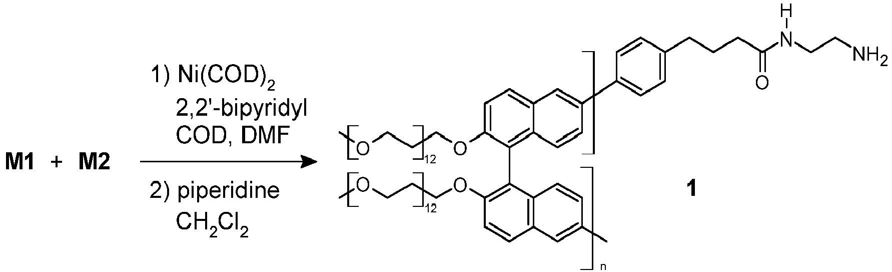

Bis(1,5-cyclooctadienyl)nickel(0) (105 mg, 382 µmol), 2,2'-bipyridyl (60.0 mg 382 µmol) and 1,5-cyclooctadiene (41.0 mg, 382 µmol) were dissolved in dimethylformamide (8 mL) under argon atmosphere and stirred for 30 min at 70°C. Compound **M1** (250 mg, 173 µmol.) and **M2** (1.3 mg, 3.4 µmol) were dissolved in dimethylformamide (4 ml) and added to the reaction mixture. The solution was stirred for 3 h at 70 °C. The solvent was evaporated and the polymer was dissolved in a mixture of dichloromethane (32 mL) and piperidine (8 mL). Volatiles were removed under reduced pressure and the residue was suspended in ethanol. After centrifugation the supernatant was freeze dried to yield the product (101 mg, 45%). GPC (270 nm): Mn: 7.9 kDa, Mw: 9.7 kDa, D = 1.2.

### Synthesis of binaphthyl binaphthyl phenylene copolymer 2

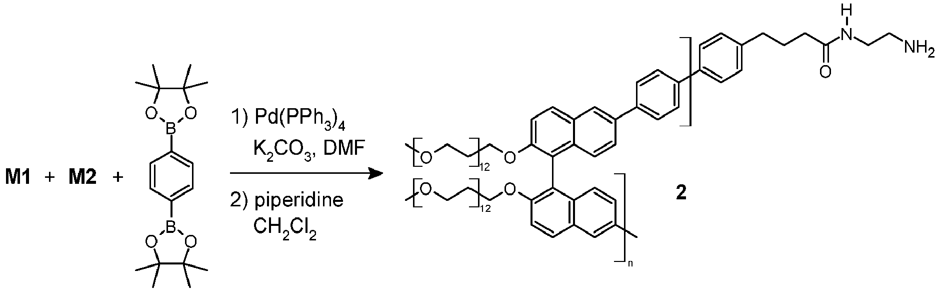

To a mixture of compound **M1** (250 mg, 173 µmol.) and 1,4-benzenediboronic acid bis(pinacol) ester (57.0 mg, 173 µmol) in dimethylformamide (4 mL) was added tetrakis(triphenylphosphine)palladium(0) (10 mg, 8.6 µmol) under argon atmosphere. Aq. potassium carbonate (2 M, 750 µL) was added and the mixture was stirred at 80 °C for 3 h. Compound **M2** (52.0 mg, 100 µmol) was added and the solvent was evaporated. The residue was suspended in 20 vol-% aq. ethanol. After centrifugation, the supernatant was freeze dried to yield the amber polymer. The polymer was purified by size exclusion purification and subsequently freeze dried. The polymer was dissolved in a mixture of dichloromethane (32 mL) and piperidine (8 mL). After evaporation of the solvent, the polymer was dissolved in 20 vol-% aq. ethanol and desalted via size exclusion purification to give the pure product (282 mg, 94.4 %). GPC (270 nm): Mn = 20.6 kDa, Mw = 34.2 kDa, D = 1.7.

### Synthesis of Binaphthyl bitiophene copolymer 3

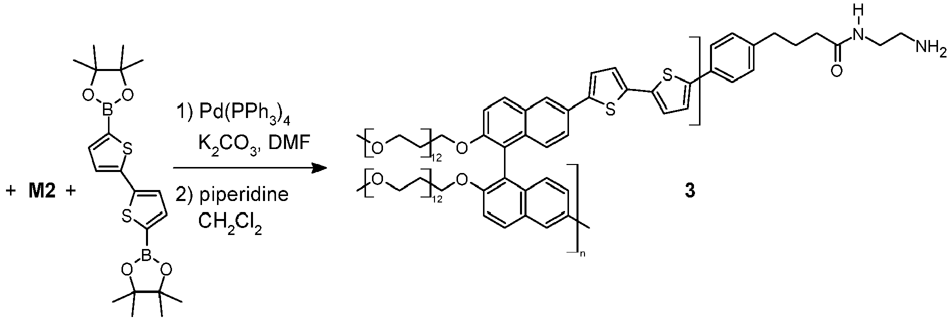

To a mixture of compound **M1** (250 mg, 173 µmol.) and 2,2'-bithiophene-5,5'-diboronic acid bis(pinacol) ester (67.0 mg 174 µmol) in dimethylformamide (4 mL) was added tetrakis(triphenylphosphine) palladium(0) (10 mg, 8.6 µmol, 0.050 eq.). After addition of aq. potassium carbonate (2 M, 750 µL), the solution was stirred at 80 °C for 3 h under argon. The solvent was evaporated and the polymer was dissolved in a mixture of dichloromethane (32 mL) and piperidine (8 mL). Volatiles were removed under reduced pressure, and the residue was suspended in 20 vol-% aq. ethanol. After centrifugation the supernatant was freeze dried to yield the amber polymer (114 mg, 46 %). GPC (270 nm): Mn: 10.7 kDa; Mw: 19,1kDa; D = 1.8.

### Bioconjugation of polymer dyes to antibodies

The antibody was reduced using dithiothreitol (50 mM) in MES buffer (pH 6) for 30 min at an antibody concentration of 5 mg/mL. The reduced antibody was purified by gel filtration oven a Sephadex G-25 column (Cytiva Europe GmbH, Germany). The antibody containing fractions were identified by Coomassie stain and the antibody concentration of the pooled fractions was determined by measuring absorption at 280 nm.

The amino-functionalized polymer was coupled to the reduced antibody using succinimidyl 4-(N-maleimidomethyl)cyclohexane-1-carboxylate (SMCC) as heterobifunctional crosslinker. The crosscoupling was carried out using a commercial SMCC-crosscoupling kit (Thermo Fisher Scientific Inc., USA). The coupling was carried out following the kit user instructions. The antibody-polymer conjugates were purified by size exclusion chromatography.

### Cell Staining

Cells were washed by vortexing at a concentration of 1 ^{∗} 10⁷ cells per mL using PEB buffer and subsequent centrifugation at 300 g for 5 min. 1 ^{∗} 10⁷ cells were resuspended in 20 µL PEB buffer, the antibody-dye conjugate and PEB buffer were added to reach a concentration of 1 ^{∗} 10⁷ cells per mL for the cell staining. The cells were stained by incubation for 10 min at 4 °C. Afterwards the cells were washed again and resuspended in 1 mL PEB buffer for flow cytometric analysis. The stained cells were stored at 4 °C until analysis. For dead cell discrimination propidium iodide solution (titer 1:1000) was added by the instrument immediately before the measurement.

### Flow Cytometric Analysis

Stained cells were analyzed by flow cytometry using a LSR Fortessa (Becton, Dickinson & Company Inc, USA) with five laser excitation sources (355 nm, 405 nm, 488 nm, 561 nm, 640 nm) in combination with a 379/28 nm bandpass filter. Flow cytometry data analysis was performed using FlowJo (Becton, Dickinson & Company Inc, USA) flow cytometry analysis software.

### Discussion

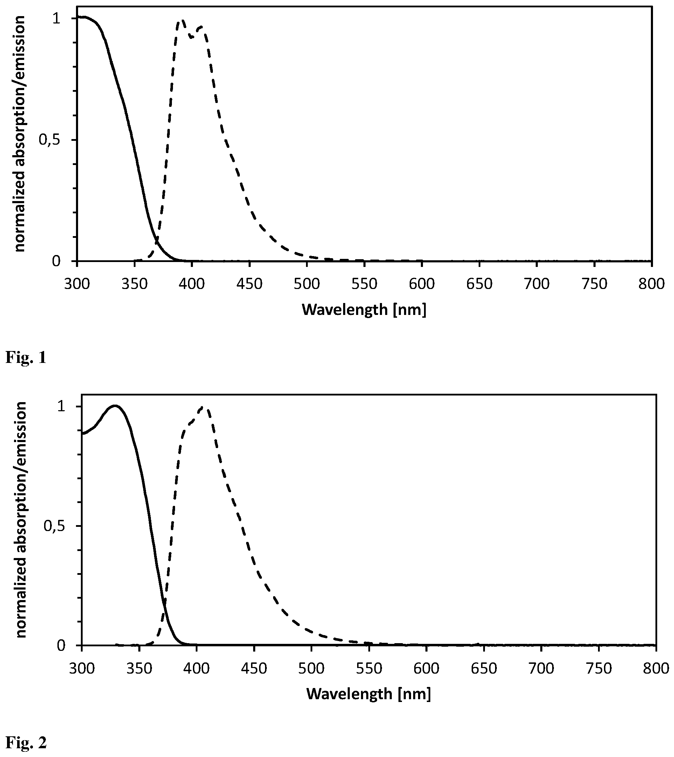

Figure 1 shows the absorption and emission spectra of polybinaphthyl homopolymer 1. The absorption and emission maxima are 315 nm and 408 nm, respectively (Table 1).

As shown in Figure 2, copolymerization of the binaphthyl monomer with phenylene monomer (copolymer **2**) resulted in a bathochromic shift of the absorption maximum by 15 nm compared to homopolymer **1**. The emission maxima of homopolymer **1** and phenylene copolymer **2** are very close (408 nm and 406 nm, respectively, see Table 1).

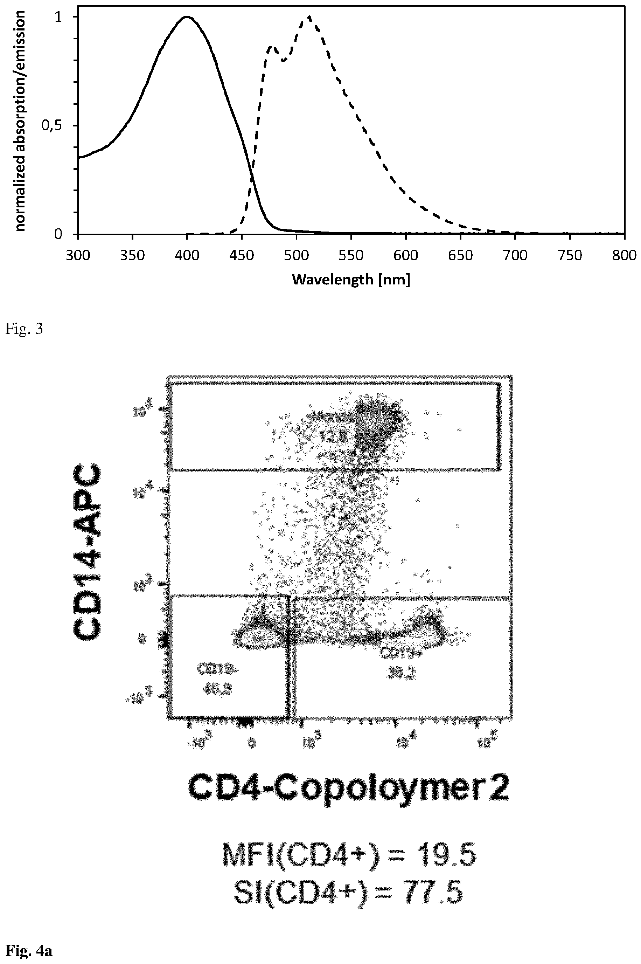

As shown in Figure 3, copolymerization of the binaphthyl monomer with a bithiophene monomer (copolymer 3) resulted in a bathochromic shift of the absorption maximum by 85 nm compared to homopolymer 1. The emission maximum of bithiophene copolymer 3 is shifted by 103 nm towards longer wavelengths compared to homopolymer 1 (Table 1).

**Table 1: Properties of water-soluble binaphthyl based polymers determined as solution in PBS buffer (pH 7.4).**

| | Mn [kDa] | Mw [kDa] | λabs. max. [nm] | λem. max. [nm] | Fl. QY |
|---|---|---|---|---|---|
| Binaphthyl homopolymer 1 | 7.9 | 9.7 | 315 | 408 | 0.60 |
| Binaphthyl phenylene copolymer (2) | 19.1 | 33.8 | 330 | 406 | 0.33 |
| Binaphthyl bitiophene copolymer (3) | 10 | 19 | 400 | 511 | 0.18 |

In the table, Fl. QY describes the fluorescence quantum yield of the synthesized polymer, which is a unit measurement of the efficiency of fluorescence conversion by the polymer in which a value of 1.00 represents 100% conversion efficiency of absorbed light photons into emitted fluorescence photons, and 0.00 represents 0% conversion. Mn (kDa) refers to the number averaged molecular weight, and Mw (kDa) refers to the mass average molecular weight.

Compound 1, 2 and 3 demonstrate that the absorption wavelength of water-soluble binaphthyl based polymers can be tuned by copolymerization with different comonomers. This invention can be used to shift the absorption band to match the wavelength of commonly used excitation laser. For example, polymer 1 (pure binaphthyl polymer) is tailored best to the Deep UV laser at 280nm; polymer 2 (Phenylene comonomer) is best suited for the UV laser at 355 nm, and polymer 3 (bisthiophene comonomer) is best suited for the Violet laser (405 nm).

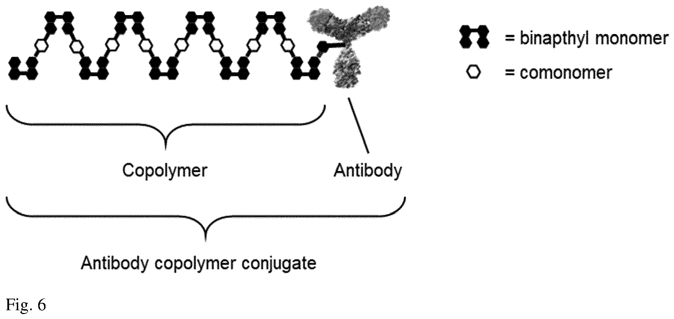

To show the usefulness of this invention, binaphthyl phenylene copolymer **2** was conjugated to a human anti-CD4 and human anti-CD19monoclonal antibody. The conjugate had the structure shown in Fig. 6.

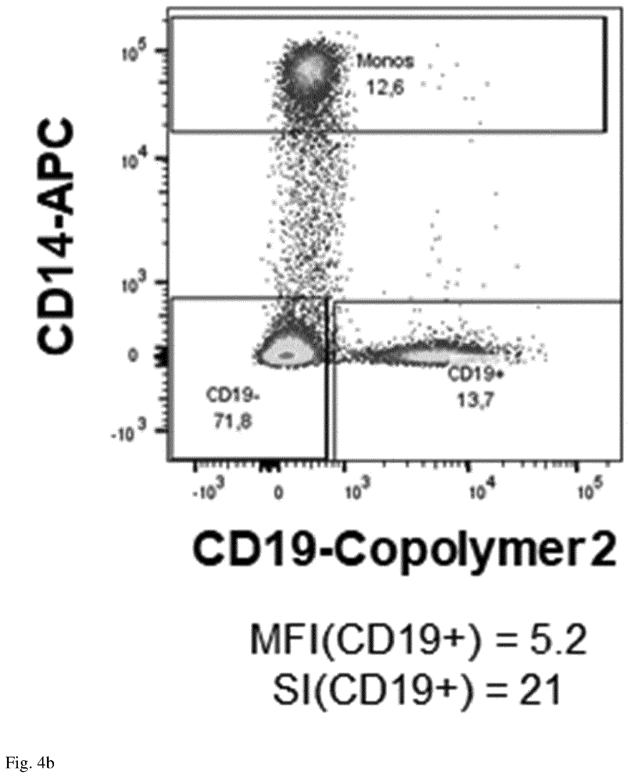

Anti-CD4 and anti-CD19 antibody conjugates of copolymer 2 were used in flow cytometric analysis of human peripheral blood mononuclear cells (PBMCs) using an UV laser (355 nm) as excitation source. Fig. 4 shows the performance of phenylene copolymer 2 antibody conjugates in flow cytometric analysis using a UV laser. To match other excitation wavelengths, the absorption wavelength can be tuned depending on the choice of the comonomer. For example, bithiophene copolymer **3** can be used for applications with the violet laser (405 nm).

These examples show the usefulness of the invention which allows to control the position of the absorption band in the spectrum to exactly match the desired excitation laser and thereby allows the expansion of the arsenal of water-soluble fluorescent polymeric dyes for biological applications.

## Claims

1. A conjugate having the general formula (I) 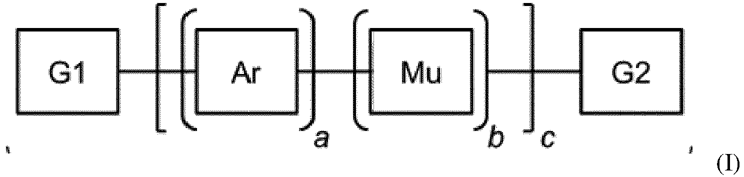
Wherein AR and MU are repeating units of a polymer
MU is a band gap modifying unit that is evenly or randomly distributed along the polymer main chain,
G1 and G2 stand for hydrogen, halogen or an antigen recognizing moiety, with the provision that at least one of G1 or G2 is an antigen recognizing moiety,
a is 10 to 100 mol%,
b is 0 to 90 mol%
c is 1 to 10 000; with the provisio that a + b = 100 mol%
**characterized in that** AR is connected in the polymer chain via the 2,2' or 3,3' or 5,5' or 6,6' or 7,7' or 8,8' positions according to general formula (II) 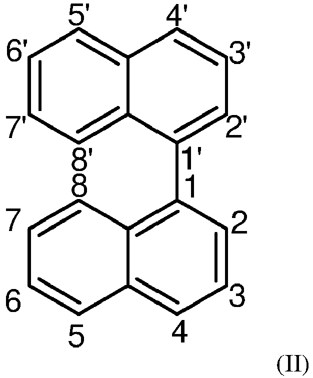
Wherein the remaining positions 2,2'; 3,3'; 4,4'; 5,5'; 6,6'; 7,7' and 8,8' are substituted with same or different residues selected from the group consisting of H, SO₂CF₃, SO₂Rₐ, CF₃, CCl₃, CN, SO₃H, NO₂, NRₐR_{b}R_{c}⁺, CHO, CORa, CO₂Ra, COCl, CONRaRb, F, Cl, Br, I, Rₐ, ORₐ, SRₐ, OCORₐ, NRₐR_{b}, NHCORₐ, CCRₐ, aryl-, heteroaryl-, C₆H₄ORₐ or C₆H₄NRaRb, with Ra-c independently hydrogen, alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or a heteroaralkyl residue or (CH₂)ₓ(OCH₂CH₂)_{y}O(CH₂)_{z}CH₃, wherein x is an integer from 0 to 20; y is an integer from 0 to 50 and z is an integer from 0 to 20

2. Conjugate according to claim 1 **characterized in that** the MU unit located on the end of the conjugate is bond via a linker group L according to formula (III) 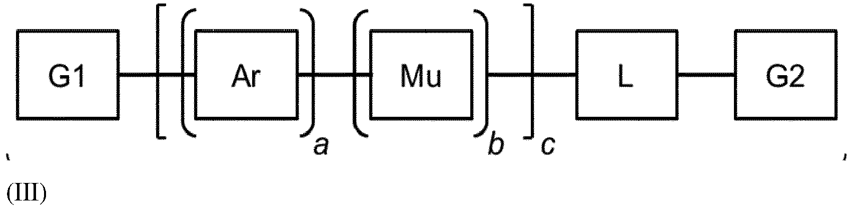 Wherein G1, AR, MU, G2, a, b and c have the same meaning as in formula (I) and wherein L represents a molecule comprising alkyl, aryl or heteroaryl

3. Conjugate according to claim 1 **characterized in that** the conjugate has a main chirality with AR provided mainly as *R*- or *S*-stereo isomer.

4. Conjugate according to any of the claims 1 to 3, **characterized in that** MU comprises an aromatic system with at least one benzene or thiophene ring providing an conjugated pi system.

5. Conjugate according to any of the claims 1 to 4, **characterized in that** MU is selected from one or more of the following residues

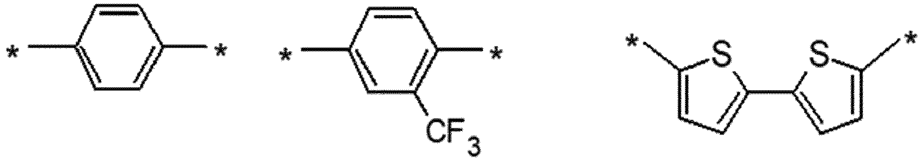

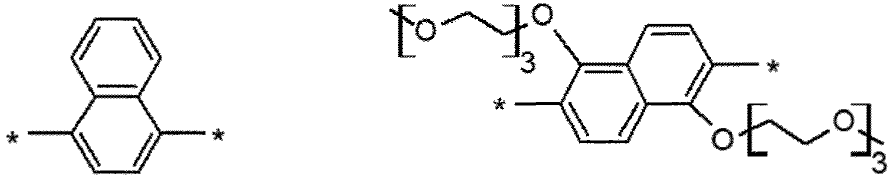

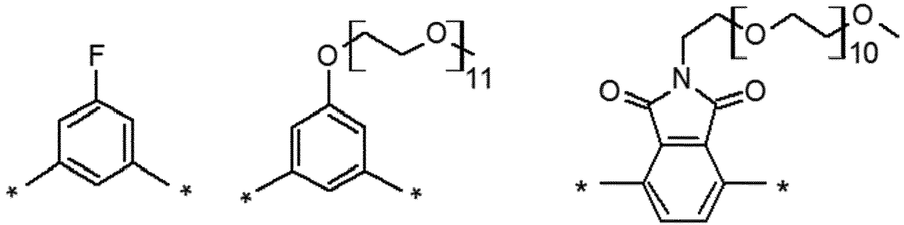

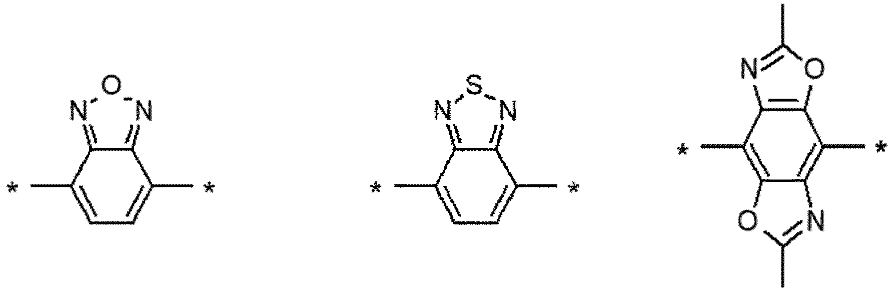

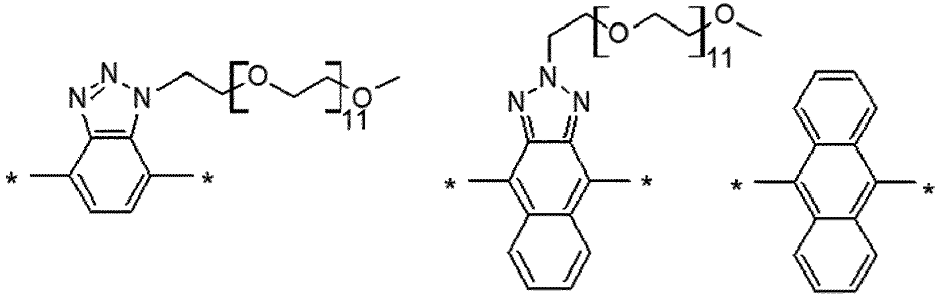

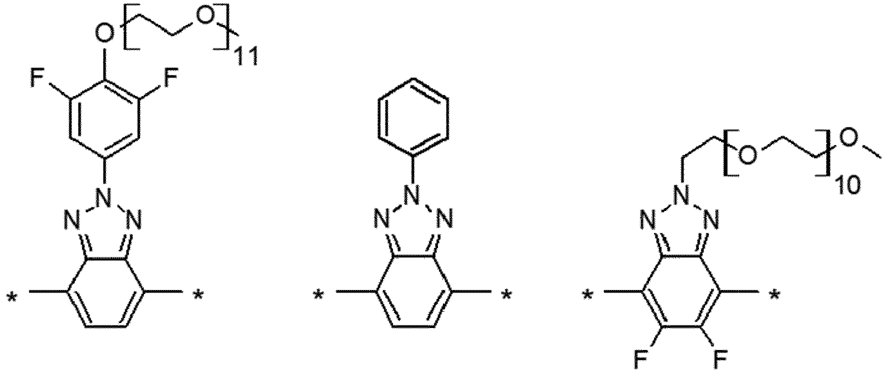

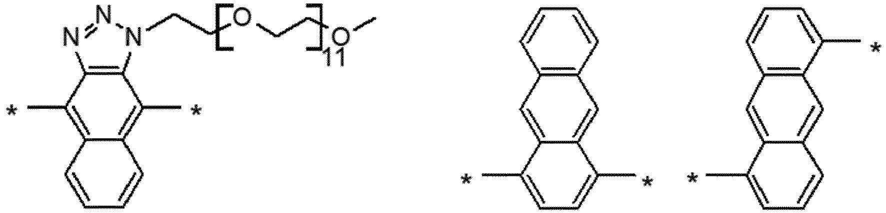

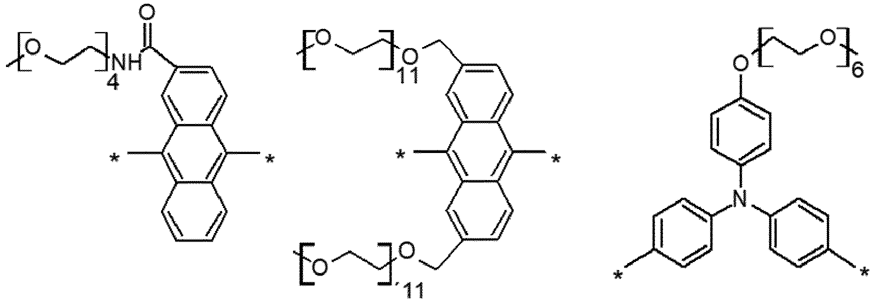

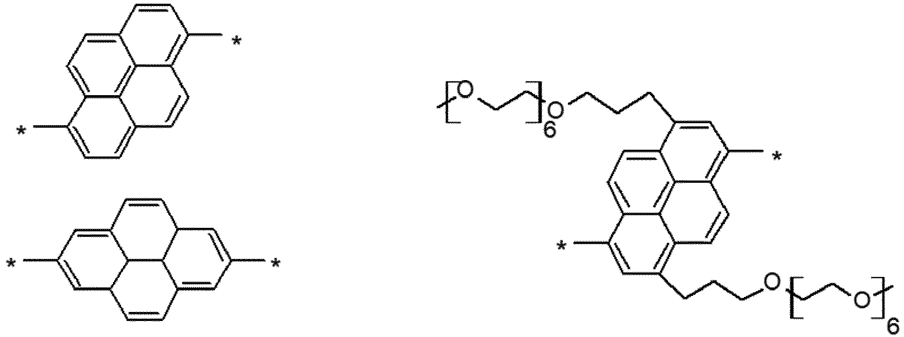

6. Conjugate according to any of the claims 1 to 5, **characterized in that** L is an aryl or a heteroaryl group located at the ends of the polymer main chain and may be substituted with one or more pendant chains terminated with: i) a functional group selected from amine, carbamate, carboxylic acid, carboxylate, maleimide, activated ester, N-hydroxysuccinimidyl, hydrazine, hydrazide, hydrazine, azide, alkyne, aldehyde, thiol, and protected groups thereof for conjugation to a molecule or biomolecule; or ii) an attached organic fluorescent dye as energy acceptor of an energy transfer system, or iii) a biomolecule.

7. The conjugate according to claim 6, **characterized in that** L is selected from one or more of the following residues:

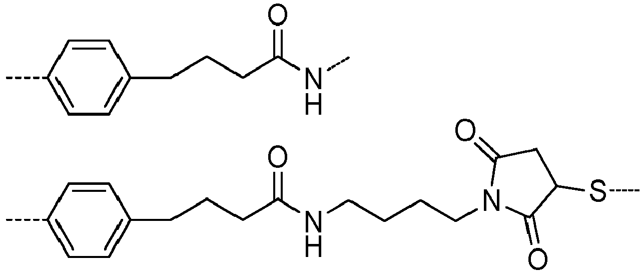

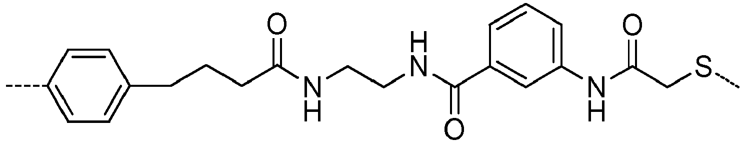

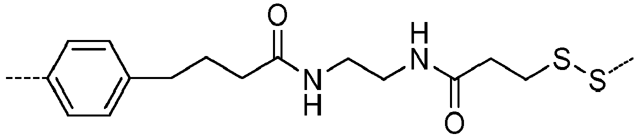

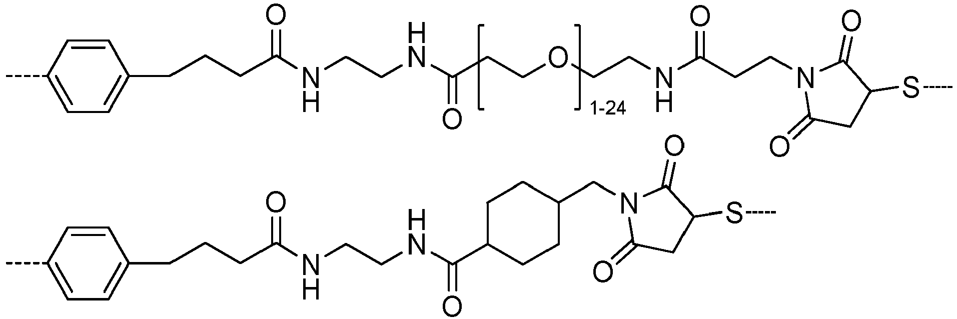

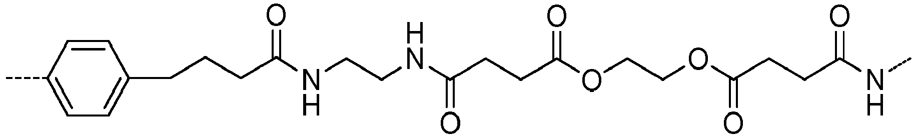

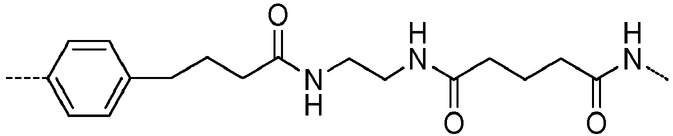

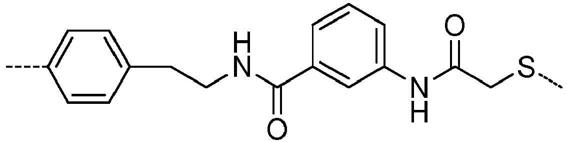

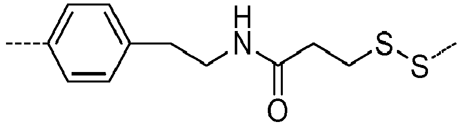

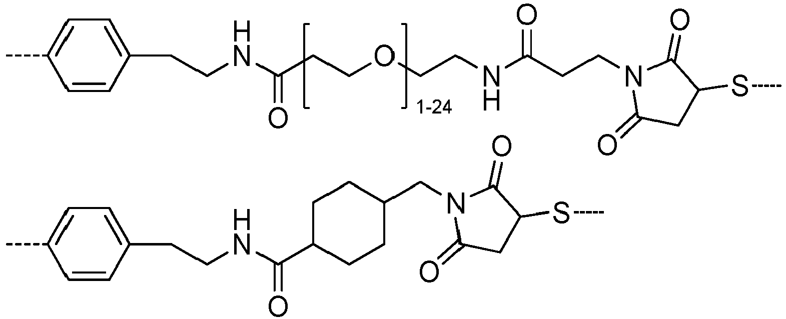

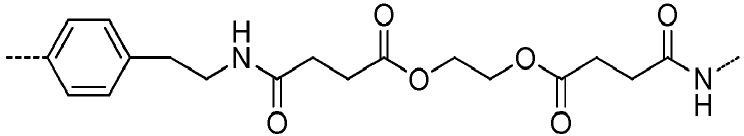

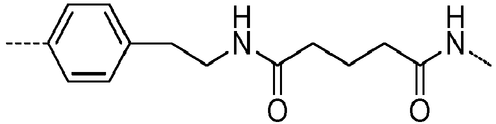

8. Conjugate according to any of the claims 1 to 7, **characterized in that** at least two positions 2,2'; 3,3'; 4,4'; 5,5'; 6,6' 7,7' and 8,8' are substituted with residues according to general formula (III) 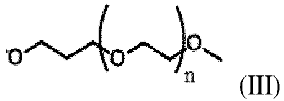 With n = 5 to 15

9. Conjugate according to any of the claims 1 to 8, **characterized in that** G1 and G2 are independently a hydrogen, halogen or an antigen recognizing moiety.

10. Conjugate according to claim 9, **characterized in that** the antigen recognizing moiety is selected from the group consisting of an antibody, an fragmented antibody, an fragmented antibody derivative, peptide/MHC-complexes, receptors for cell adhesion or costimulatory molecules, receptor ligands, antigens, hapten binders, avidin, streptavidin, aptamers, primers and ligase substrates, peptide/MHC complexes targeting TCR molecules, cell adhesion receptor molecules, receptors for costimulatory molecules or artificial engineered binding molecules.

11. Method for detecting a target moiety in a sample of biological specimens with at least one conjugate according to any of the claims 1 to 10 **characterized by** the steps
d) contacting the sample of biological specimens with at least one conjugate, thereby labeling the target moiety recognized by an antigen recognizing moiety with the conjugate;
e) exciting the labelled target moieties with light having a wavelength within the absorbance spectrum of the conjugate
f) detecting the labelled target moieties by detecting the fluorescence radiation emitted by the conjugate.

12. Use of the conjugate according to any of the claims 1 to 10 in fluorescence microscopy, flow cytometer, fluorescence spectroscopy, cell separation, pathology or histology.
